# EUROPEAN PATENT APPLICATION

(11) **EP 3 409 291 A1**
(43) Date of publication of application: **05.12.2018**
(21) Application number: 17744444.5
(22) Date of filing: 27.01.2017
(51) Int. Cl.: A61K 47/08, A61K 9/08, A61K 47/06, A61K 47/10, A61K 47/14, A61K 47/34, A61K 47/44

(54) **TRANSDERMALLY ABSORBABLE COMPOSITION HAVING CONTROLLED RELEASE OF WATER-SOLUBLE ACTIVE INGREDIENT**

(30) Priority: 29.01.2016 JP 2016015973
(71) Applicant: Nissan Chemical Corporation, Tokyo 103-6119 (JP); Kyushu University, National University Corporation, Fukuoka-shi Fukuoka 812-8581 (JP)
(72) Inventor: ONO, Fumiyasu, Fukuoka-shi Fukuoka 812-8581 (JP); GOTO, Masahiro, Fukuoka-shi Fukuoka 812-8581 (JP); HIRATA, Osamu, Funabashi-shi Chiba 274-0052 (JP); IWAMA, Takehisa, Funabashi-shi Chiba 274-0052 (JP)
(74) Representative: dompatent von Kreisler Selting Werner - Partnerschaft von Patent- und Rechtsanwälten mbB
(86) International application number: PCT/JP2017/003067
(87) International publication number: WO 2017/131214

(57) **Abstract**

The present invention addresses the problem of providing a method for increasing the transdermal absorption of a water-soluble drug in a simple manner without relying on the formation of a prodrug of the water-soluble drug by organic synthesis. The present invention relates to: a complex which is formed from a water-soluble active ingredient and a fat-soluble compound having an aldehyde group through an interaction capable of being dissociated in water; a transdermally absorbable composition comprising the complex, a transdermal absorption controlling agent and a fat-soluble medium; and a method for controlling the release of the water-soluble active ingredient from the complex in skin.

## Description

### TECHNICAL FIELD

The present invention relates to a percutaneously absorbable composition controlled in release of a water-soluble effective ingredient in the skin, and a method for controlling the water-soluble effective ingredient in the skin.

### BACKGROUND ART

In recent years, studies on percutaneous absorption techniques which induce active ingredients to be absorbed from the skin to act directly on the skin are progressing.

In particular, with respect to water-soluble drugs, it is a problem that it is difficult to penetrate into a stratum corneum having high fat-solubility, and as one method for enhancing the percutaneous absorption thereof, it is mentioned fat solubilization (oil solubilization) of the water-soluble drug. For example, a lipophilic site is introduced (by making prodrug) to a water-soluble drug by organic synthesis, dissolved and dispersed in an oleaginous base material and applied to the skin. After permeating to the stratum corneum, the bond between the water-soluble drug and the fat-soluble portion is cut by enzymes, etc., inside the skin, and the water-soluble drug is released. Alternatively, an aqueous solution of a water-soluble drug is encapsulated in liposomes, dissolved and dispersed in an oleaginous base material and applied to the skin. After permeating to the stratum corneum, the interface of the liposome is disintegrated inside the skin by enzymes, etc., and the water-soluble drug is released.

Recently, it has been reported that a permeability enhancer (percutaneous absorption promoter) such as a terpene or a higher alcohol, etc., is used for enhancing percutaneous absorbability of a drug (Patent Documents 1 to 5).

### PRIOR ART DOCUMENTS

### Patent Documents

Patent document 1: JP 2013-241459A
Patent document 2: WO 2012/043701A
Patent document 3: JP 2010-100650A
Patent document 4: JP 5,680,197C
Patent document 5: JP 2010-6771A

### SUMMARY OF THE INVENTION

### Problems to be Solved by the Invention

Although prodrug formation is an excellent method of imparting lipophilicity to a water-soluble drug and enhancing percutaneous absorbability, it is required to take a manufacturing process of organic synthesis and a complicated procedure for obtaining approval for producing and marketing of pharmaceuticals and quasi-drugs.

On the other hand, as a drug to be made into a prodrug or encapsulated in a liposome, for example, it has been known that tranexamic acid which is an artificial amino acid binds to plasmin as a proteolytic enzyme in the epidermis and inhibits activation thereof, thereby suppressing the production of melanin. By utilizing this function, it has been demonstrated its skin whitening effects in recent years, and attracting attention as a raw material for cosmetics. However, there was a problem that skin permeability of the tranexamic acid was low.

It is an object of the present invention to provide a method for simply and conveniently enhancing the percutaneous absorbability of a water-soluble drug without using prodrug formation by organic synthesis.

### Means to Solve the Problems

The present inventors have found, as a result of intensive studies, that a water-soluble effective ingredient forms a complex with a fat-soluble compound having an aldehyde group through a reversible interaction that can be dissociated in water, so that fat-solubility is provided to the water-soluble effective ingredient simply and conveniently to enhance its percutaneous absorbability while on the other hand the water-soluble effective ingredient can be easily released in the skin, whereby they have accomplished the present invention.

That is, the present invention relates to
(1) a complex formed by a water-soluble effective ingredient and a fat-soluble compound having an aldehyde group through an interaction which can be dissociated in water.
(2) the complex described in the (1), wherein the fat-soluble compound having an aldehyde group is at least one selected from the group consisting of terpene having an aldehyde group, lignoid having an aldehyde group, and vanilloid having an aldehyde group;
(3) the complex described in the (2), wherein the terpene having an aldehyde group is citral or citronellal;
(4) the complex described in the (2), wherein the lignoid having an aldehyde group is cinnamaldehyde;
(5) the complex described in the (2), wherein the vanilloid having an aldehyde group is vanillin;
(6) the complex described in any one of the (1) to (5), wherein the water-soluble effective ingredient is a compound having an amino group, a hydroxyl group or a thiol group;
(7) a percutaneously absorbable composition which comprises the complex described in any one of the (1) to (6), a percutaneous absorption controller and a fat-soluble medium;
(8) the percutaneously absorbable composition described in the (7), wherein the complex is dissolved in the fat-soluble medium;
(9) the composition described in the (7) or (8), wherein the fat-soluble medium is a percutaneous absorption promotable fat-soluble medium;
(10) the composition described in the (9), wherein the percutaneous absorption promotable fat-soluble medium is one or more selected from the group consisting of isopropyl myristate, squalane, squalene, silicone oil, jojoba oil, almond oil, olive oil, horse oil, and mineral oil;
(11) a composition which comprises a water-soluble effective ingredient, a fat-soluble compound having an aldehyde group and a percutaneous absorption controller;
(12) the composition described in the (11), which further comprises a fat-soluble medium;
(13) the composition described in any one of the (7) to (12), wherein the percutaneous absorption controller is an alcohol;
(14) the composition described in the (13), wherein the alcohol is at least one selected from the group consisting of methanol, ethanol, geraniol, isopropyl alcohol and glycerol;
(15) a method for controlling release of the water-soluble effective ingredient from the complex described in any one of the (1) to (6) in skin which comprises a step of dissolving the complex in a percutaneous absorption controller;
(16) a method for releasing the water-soluble effective ingredient from the complex described in any one of the (1) to (6) in skin which comprises a step of dissolving the complex in a percutaneous absorption controller;
(17) a method for releasing a water-soluble effective ingredient from a composition containing a water-soluble effective ingredient, a fat-soluble compound having an aldehyde group and a percutaneous absorption controller in skin;
(18) a method of producing a complex formed by a water-soluble effective ingredient and a fat-soluble compound having an aldehyde group through an interaction which can be dissociated in water, which comprises a step of mixing powder of the water-soluble effective ingredient and the fat-soluble compound having an aldehyde group;
(19) a method for producing a composition containing a water-soluble effective ingredient, a fat-soluble compound having an aldehyde group and a percutaneous absorption controller, which comprises a step of mixing powder of the water-soluble effective ingredient, the fat-soluble compound having an aldehyde group and the percutaneous absorption controller;
(20) the complex described in the (1), wherein the fat-soluble compound having an aldehyde group is contained in the essential oil; and
(21) the complex described in (20), wherein the essential oil is lemon grass or citronella.

### Effects of the Invention

In the complex of the present invention, the water-soluble effective ingredient and the fat-soluble compound having an aldehyde group are compounded through an interaction capable of dissociating in water, so that it can be uniformly dissolved (fat-solubilized, or oil-solubilized) in a fat-soluble medium, and as a result, percutaneous absorbability of the water-soluble effective ingredient can be improved, and after permeation to the stratum corneum, the water-soluble effective ingredient is released by water inside the skin and the medicinal effect can be also accomplished.

Also, in the present invention, release of the water-soluble effective ingredient from the complex in the skin can be also controlled by appropriately using a percutaneous absorption controller.

### BRIEF DESCRIPTION OF THE DRAWINGS

Fig. 1 is a photograph showing appearance of the percutaneously absorbable compositions of Comparative examples 1 to 4 and Example 2.
Fig. 2 is a photograph showing appearance of the percutaneously absorbable compositions of Comparative example 5 (A in the drawing), Example 7 (B in the drawing) and Example 8 (C in the drawing).
Fig. 3 is a photograph showing appearance of the percutaneously absorbable compositions of Comparative example 6 (A in the drawing), Example 13 (B in the drawing) and Example 14 (C in the drawing).
Fig. 4 is a photograph showing appearance of the tranexamic acid-(-)-citronellal complex in an ethanol solution (A in the drawing), an ethyl acetate solution (B in the drawing) and a hexane solution (C in the drawing).
Fig. 5 is a drawing showing an ESI-TOF-MASS spectrum of the tranexamic acid-(-)-citronellal complex in an ethanol solution.
Fig. 6 is a drawing showing an ESI-TOF-MASS spectrum of the tranexamic acid-(-)-citronellal complex in an ethyl acetate solution.
Fig. 7 is a drawing showing an ESI-TOF-MASS spectrum of the tranexamic acid-(-)-citronellal complex in a hexane solution.

### EMBODIMENTS TO CARRY OUT THE INVENTION

In the complex of the present invention, the water-soluble effective ingredient and the fat-soluble compound having an aldehyde group are compounded through an interaction which can be dissociated in water. Therefore, the complex of the present invention can be in a chemical equilibrium relationship with the water-soluble effective ingredient and the fat-soluble compound having an aldehyde group, and as a result, in the fat-soluble medium, it can be fat-solubilized to be stably present by solvation or formation of cluster, etc., and on the other hand, inside the skin, it easily dissociates with moisture contained therein and releases the water-soluble effective ingredient, so that it can exert its medicinal effect.

Accordingly, the water-soluble effective ingredient of the present invention is not particularly limited as long as it is a water-soluble effective ingredient for a medicine or a cosmetic product which is capable of forming a complex with a fat-soluble compound having an aldehyde group through an interaction capable of dissociating in water, and any water-soluble effective ingredient for a medicine or a cosmetic product, for example, an amino acid, hydrophilic vitamin, sugar, peptide and other hydrophilic drugs, etc., can be used.

As examples of such a water-soluble effective ingredient of the present invention, those having an amino group, a hydroxyl group or a thiol group are mentioned. Each of these groups can form an interaction capable of dissociating in water, for example, an ionic bond, a hydrogen bond, a dipole interaction, a van der Waals force, a charge transfer interaction, π-π interactions, hydrophobic interactions or solvation, reversible chemical bonds, etc. with an aldehyde group of a fat-soluble compound having the aldehyde group,

As examples of the amino acid, there are mentioned artificial amino acids such as tranexamic acid, etc.; and natural amino acids such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine, etc.

As examples of the hydrophilic vitamin, there are mentioned ascorbic acid, sodium ascorbyl phosphate, magnesium ascorbyl phosphate, ascorbyl glucoside, ethyl ascorbic acid and vitamins B1, B2, B4, B5, B6, B7 and B12, etc.

As examples of the sugar, there are mentioned glucose, trehalose, dextran, pullulan, cyclodextrin, mannitol, glucosamine, galactosamine, raffinose, mannan and pectin, etc.

As the peptide, there are mentioned a peptide having a specific efficacy (for example, expected to be effective in treating pollen allergy because they are recognized by human T cells, etc.) in addition to the known peptides. Specific examples thereof are Peptide A (amino acid sequence: QFAKLTGFTLMG) and Peptide B (amino acid sequence: SMKVTVAFNQFGP).

As examples of the hydrophilic drugs, there are mentioned minoxidil, zanamivir (relenza), aciclovir, cytarabine oxephosphate and fludarabine phosphate, etc.

In the present invention, the water-soluble effective ingredient may be used alone or in combination of two or more kinds thereof.

The fat-soluble compound having an aldehyde group of the present invention is not particularly limited as long as it is a fat-soluble compound having at least one aldehyde group, and any compound including aliphatic aldehyde or aromatic aldehyde can be used. Among them, a terpene having an aldehyde group, a lignoid having an aldehyde group and a vanilloid having an aldehyde group, etc., are preferable from the viewpoint of natural origin and safety.

As examples of the terpene having an aldehyde group, there are mentioned citral, citronellal, cyclocitral, safranal, phellandral, perillaldehyde, tagetone and retinal, etc., from the viewpoint of satisfactorily fat-solubilizing the water-soluble effective ingredient, citral and citronellal are preferable, and natural products such as essential oil (essential oils), etc., containing the above compounds may be used. Lemon grass or citronella is preferred from the viewpoint of satisfactorily fat-solubilizing the water-soluble effective ingredient.

Essential oil is a generic term for volatile organic substances produced by plants, and is generally a mixture of many compounds. It is not particularly limited, and it can be carried out as long as it contains a terpene having an aldehyde group of the present invention.

Lemon grass is, as an example, a mixture containing citral (50 to 80%) as a main component, geraniol (3 to 10%), farnesol, nerol, citronellol and myrcene, etc. Citronella is, as an example, a mixture containing geraniol as a main component (10 to 60%), citronellol (3 to 10%), citronellal (1 to 30%), etc.

Cinnamaldehyde is preferred as the lignoid having an aldehyde group from the viewpoint of satisfactorily fat-solubilizing the water-soluble effective ingredient.

Vanillin is preferred as the vanilloid having an aldehyde group from the viewpoint of satisfactorily fat-solubilizing the water-soluble effective ingredient.

As the aliphatic aldehyde or aromatic aldehyde, dodecanal or 4-butoxybenzaldehyde is also preferable from the viewpoint of satisfactorily fat-solubilizing the water-soluble effective ingredient.

In the present invention, the fat-soluble compound having an aldehyde group may be used alone or in combination of two or more kinds thereof.

The fat-soluble compound having an aldehyde group of the present invention is particularly preferably citral and citronellal from the viewpoint of satisfactorily fat-solubilizing the water-soluble effective ingredient.

The percutaneous absorption controller of the present invention is not particularly limited as long as it is commonly used in controlling percutaneous absorption of active ingredients of water-soluble medicines or cosmetics in the fields of pharmaceuticals and cosmetics, in particular, in the fields of the external preparation for skin. A percutaneous absorption controller having a hydroxyl group is preferable from the viewpoint that the complex of the present invention can be favorably stabilized in a fat-soluble medium, particularly a monohydric or polyhydric alcohol is preferably used.

In the complex of the present invention, in addition to the water-soluble effective ingredient and the fat-soluble compound having an aldehyde group, the percutaneous absorption controller may further be compounded through an interaction capable of dissociation in water. In such a complex, release of the water-soluble effective ingredient in the skin is better controlled.

As examples of the monohydric alcohol, there are mentioned a lower alcohol such as methanol, ethanol, 1-propanol and 2-propanol, etc.; a higher alcohol; and a terpene having a hydroxyl group such as geraniol, menthol, borneol, isoborneol, nellol, citronellol, fenchyl alcohol, carveol and neomenthol, etc., and preferably methanol, ethanol and geraniol.

As examples of the higher alcohol, there are mentioned a saturated alcohol having 8 to 18 carbon atoms such as octanol, nonanol, decanol, undecyl alcohol, lauryl alcohol, tridecyl alcohol, myristyl alcohol, pentadecyl alcohol, cetyl alcohol, heptadecyl alcohol and stearyl alcohol, etc.; and an unsaturated alcohol having 8 to 18 carbon atoms such as oleyl alcohol, linoleyl alcohol, and linolenyl alcohol, etc. Preferably mentioned are octanol, decanol, lauryl alcohol, myristyl alcohol and oleyl alcohol, and more preferably mentioned is oleyl alcohol.

As examples of the polyhydric alcohol, there are mentioned ethylene glycol, propylene glycol, 1,3-propanediol, 1,2-butanediol, 1,3-butanediol, 1,4-butanediol, 2,3-butanediol, 1,5-pentanediol, glycerin, dipropylene glycol, diethylene glycol, triethylene glycol and polyethylene glycol 400, etc., and preferably there are mentioned propylene glycol.

The complex of the present invention can be simply and conveniently prepared by mixing the water-soluble effective ingredient and the fat-soluble compound having an aldehyde group, and heating and cooling for a certain period of time, if necessary. It is preferable to add and mix a percutaneous absorption controller of the present invention, in particular, a liquid state percutaneous absorption controller from the viewpoint of obtaining a complex well. In addition, the percutaneous absorption controller of the present invention is preferably in a liquid state also from the viewpoint of the obtained complex being stably dissolved. In this case, the solution of the percutaneous absorption controller in which the obtained complex is dissolved can be used for preparation of the percutaneously absorbable composition of the present invention.

In the case where the water-soluble effective ingredient is a solid (preferably a crystal of amino acid), it is preferable to mix it with powder state in order to obtain the complex of the present invention well. As a method of making it into powder, a method by dry pulverization is mentioned. The conditions for the dry pulverization are not particularly limited as long as it is a condition capable of pulverizing the water-soluble effective ingredient into small pieces, and it is desirable to use a pulverizer such as a mortar, a ball mill, a homogenizer, a cutter mill, a hammer mill, etc. In addition, the pulverization time, the treatment pressure, etc., are appropriately adjusted according to the hardness of the water-soluble effective ingredient to be pulverized.

It is desirable that the powder obtained by the method mentioned above is further sieved in order to make the particle size uniform. As the sieve, it is preferably 500 µm or less, more preferably 200 µm or less, and particularly preferably 100 µm or less.

A mixing ratio of the water-soluble effective ingredient and the fat-soluble compound having an aldehyde group may vary depending on the kinds of these substances to be used, and is 1:1 to 100, preferably 1:10 to 50.

In the case of also mixing the percutaneous absorption controller, a mixing ratio of the water-soluble effective ingredient, the fat-soluble compound having an aldehyde group and the percutaneous absorption controller may vary depending on the kinds of these substances to be used, and is 1:1 to 100:1 to 100, preferably 1:1 to 100:10 to 50, and more preferably 1:10 to 50:10 to 50.

A constitutional ratio (molar ratio) of the water-soluble effective ingredient and the fat-soluble compound having an aldehyde group constituting the complex of the present invention is 1 to 100:100 to 1, more preferably 1 to 10:10 to 1.

In the present invention, by using the percutaneous absorption controller mentioned above, in particular, by appropriately using a monohydric or polyhydric alcohol, for example, depending on the type or the used amount of these percutaneous absorption controllers, release of the water-soluble effective ingredient from the complex of the present invention in the skin after percutaneous absorption can be controlled. For example, it is possible to delay release of the water-soluble effective ingredient of the present invention by increasing fat solubility of the percutaneous absorption controller (for example, by increasing the carbon number), and it is possible to promote release of the water-soluble effective ingredient of the present invention by lowering fat solubility of the percutaneous absorption controller (for example, by decreasing the carbon number). The present invention also relates to a method for controlling release of the water-soluble effective ingredient from the complex of the present invention in the skin, which comprises a step of dissolving the complex in the percutaneous absorption controller. The present invention further relates to use of a percutaneous absorption controller for controlling release of the water-soluble effective ingredient from the complex of the present invention in the skin.

In the present invention, the percutaneous absorption controller may be used singly or in combination of two or more kinds.

The fat-soluble medium of the present invention is not particularly limited as long as it is a fat-soluble medium commonly used as a substrate, etc., in the field of medicine and cosmetic products, particularly in the field of external preparations for skin, and preferably a percutaneous absorption promotable fat-soluble medium can be used.

The percutaneous absorption promotable fat-soluble medium is not particularly limited as long as it is commonly used for promoting percutaneous absorption of effective ingredients of water-soluble pharmaceuticals or cosmetics in the field of medicine and cosmetics, particularly external preparations for skin, for example, it is possible to use isopropyl myristate (IPM), squalane, squalene, silicone oil, jojoba oil, almond oil, olive oil, horse oil, and mineral oil, etc., preferably to use isopropyl myristate (IPM).

In the present invention, the fat-soluble medium mentioned above may be used alone or in combination of two or more kinds thereof.

The percutaneously absorbable composition of the present invention can be prepared by mixing the above obtained solution of the percutaneous absorption controller in which the complex is dissolved and the fat-soluble medium.

The percutaneously absorbable composition of the present invention may be used per se when it is used as a medicine or a cosmetic product, and it may be used as a conventional pharmaceutical preparation, in particular, an external preparation for skin or a cosmetic product. The pharmaceutical preparation or cosmetic product may contain an additive(s) acceptable in the fields of pharmaceuticals and cosmetics such as an excipient, a lubricant, a binder, a disintegrator, an emulsifier, a stabilizer, a flavoring agent, a diluent, etc., as long as it does not impair the effects of the present invention.

The external preparation for skin of the present invention may contain a component which can be usually formulated in an external preparation for skin as long as it does not impair the effects of the present invention. As such a component, a polyhydric alcohol such as glycerin and propylene glycol, etc., oils such as liquid paraffin, squalane, higher fatty acids and higher alcohols, etc., organic acids such as citric acid and lactic acid, etc., alkalis such as caustic soda and triethanolamine, etc., a cationic surfactant, an amphoteric surfactant, a nonionic surfactant, powder, a pigment, a dye, an antiseptic and antifungal agent, a resin, a pH adjusting agent, an antioxidant, an ultraviolet absorber, a chelating agent, a thickener, a humectant, an alcohol, water, and a perfume, etc. are exemplified.

The present invention also relates to a method for percutaneously administering the water-soluble effective ingredient, which comprises applying the percutaneously absorbable composition of the present invention to the skin. In addition, the present invention also relates to use of the percutaneously absorbable composition of the present invention for percutaneously administering the water-soluble effective ingredient.

Also, the composition of the present invention may be a composition containing the water-soluble effective ingredient, the fat-soluble compound having an aldehyde group and the percutaneous absorption controller, and is preferably a percutaneously absorbable composition.

The composition of the present invention may further contain the fat-soluble medium.

The present invention also relates to a method for percutaneously administering the water-soluble effective ingredient which comprises applying the composition of the present invention to the skin. In addition, the present invention also relates to use of the composition of the present invention for percutaneously administering the water-soluble effective ingredient.

### EXAMPLES

In the following, the present invention will be explained in more detail by referring to Examples, but the present invention is not limited by these.

### 1. Reagents and apparatus

The reagents used in Examples are shown below.

Citronellol (first grade), geranic acid (mixture of isomers), ethanol (special grade), isopropyl myristate (IPM) (special grade), glycine, L-cysteine (special grade), L-serine (special grade), L(-)-threonine (special grade), L-tryptophan (special grade), L(-)-proline (special grade), L-glutamic acid (special grade), L(+)-glutamine (special grade), L-valine (special grade), L(+)-isoleucine (special grade), L-tyrosine (special grade), 10×D-PBS (-) (for cell adhesion), triethylamine (special grade), 1-dodecanal, cinnamaldehyde (special grade), sodium dodecyl sulfate (first grade), and phosphoric acid (special grade) were obtained from Wako Pure Chemical Industries, Ltd. Trans-4-(aminomethyl)cyclohexanecarboxylic acid (tranexamic acid), (-)-citronellal, citral (cis-and trans-mixture), geraniol, L-alanine, L-ascorbic acid , 4-butoxybenzaldehyde, vanillin, L-(+) lysine, L-histidine and minoxidil were obtained from Tokyo Chemical Industry Co., Ltd. Methanol (special grade) and anhydrous sodium dihydrogenphosphate (special grade) were obtained from Wako Pure Chemical Industries, Ltd. Peptide A (amino acid sequence: QFAKLTGFTLMG), and Peptide B (amino acid sequence: SMKVTVAFNQFGP) were obtained from Medical & Biological Laboratories Co., Ltd.. Lemon grass and citronella were obtained from Harvest Season Co., Ltd. (import source: Quinessence). Hexane (special grade), ethyl acetate (special grade) and acetonitrile (for high performance liquid chromatography) were obtained from Kanto Chemical Co., Inc.

The apparatuses used for the analysis are shown below.

### High performance liquid chromatography (HPLC)

Apparatus: 1200 Series, manufactured by Agilent Technologies, Inc. Mass spectrometer (ESI-TOF-MASS)
Apparatus: TOFMS system equipped with JMS-T100CS cold spray ion source, manufactured by JEOL Ltd.

### Microplate reader

Apparatus: Infinite 200PRO Series, manufactured by TEKAN

### 2. Preparation example of percutaneously absorbable composition containing tranexamic acid oil-solubilized by terpene

To 4 mL of a screw-top sample tube were added 10 mg of tranexamic acid, 100 mg of various terpenes shown in Table 1, and 0.5 mL of ethanol, and the mixture was heated at 80°C for 1 hour. After heating, the mixture was allowed to cool for 5 minutes at room temperature, 3 mL of isopropyl myristate (IPM) was added to prepare percutaneously absorbable compositions of Comparative examples 1 to 4 and Examples 1 and 2.

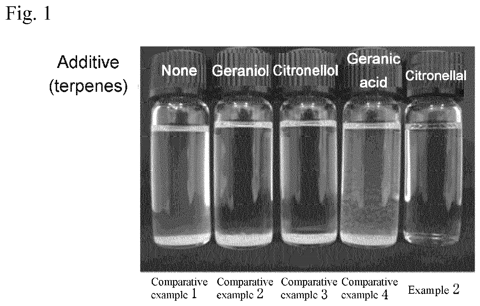

The dispersed state of these Examples and Comparative examples was observed to confirm the presence or absence of oil solubilization. The results are shown in Table 1 and Fig. 1.

### [Table 1]

**Table 1 Addition effect of terpene in fat-solubilization of tranexamic acid**

| | Comparative example 1 | Comparative example 2 | Comparative example 3 | Comparative example 4 | Example 1 | Example 2 |
|---|---|---|---|---|---|---|
| Kind of terpene | Not added | Geraniol | Citronellol | Geranic acid | Citral | (-)-Citronellal |
| Solution state | Powder precipitate | Powder precipitate | Powder precipitate | Powder precipitate | Yellow uniform solution | Pale yellow uniform solution |

From these results, it could be understood that tranexamic acid could be uniformly oil-solubilized in IPM by adding citral or citronellal having an aldehyde group among the terpenes.

Further, preparation at room temperature was carried out.

To 4 mL of a screw-top sample tube were added 20 mg of tranexamic acid, 200 mg of citronellal and 1 mL of ethanol, and the mixture was stirred at room temperature for 3 days. After 3 days, 1 mL of IPM was added. As a result, a pale yellow uniform solution was obtained similar to the case where heating at 80°C.

### 3. Preparation example of percutaneously absorbable composition containing tranexamic acid oil-solubilized by additive other than terpene

To 4 mL of a screw-top sample tube were added 10 mg of tranexamic acid, 200 mg of an additive shown in Table 2 and 1 mL of ethanol, and the mixture was heated to 80°C until a uniform solution was obtained. After heating, the mixture was allowed to cool for 5 minutes at room temperature, and 1 mL of IPM was added to prepare percutaneously absorbable compositions of Examples 3 to 6.

The dispersed state of these Examples and Comparative examples was observed to confirm the presence or absence of oil solubilization. The results are shown in Table 2.

### [Table 2]

**Table 2 Addition effect of additive in oil-solubilization of tranexamic acid**

| | Example 3 | Example 4 | Example 5 | Example 6 |
|---|---|---|---|---|
| Kind of additive | Cinnamaldehyde | Vanillin | Dodecanal | 4-Butoxybenzaldehyde |
| Heating time | 1 hour | 5 hours | 1 hour | 5 hours |
| Solution state | Yellowish brown | Yellow | Pale yellow | Pale yellow |

From these results, it could be understood that tranexamic acid could be uniformly oil-solubilized in IPM by adding cinnamaldehyde, vanillin, dodecanal and 4-butoxybenzaldehyde, which have an aldehyde group and are used in food additives, etc., other than terpene.

### 4. Preparation example of percutaneously absorbable composition containing tranexamic acid oil-solubilized by essential oil

A preparation example using lemon grass which has been known as essential oil as an oil solubilization additive of tranexamic acid is shown below.

To 4 mL of a screw-top sample tube were added 20 mg of tranexamic acid, 214 mg of lemon grass and 0.5 mL of ethanol, and the mixture was stirred for 2 days at room temperature to prepare an ethanol solution. Two days later, to 4 mL of a screw-top sample tube separately prepared were added 0.1 mL of the ethanol solution prepared above and 0.9 mL of IPM. As a result, a red brown uniform solution was obtained.

Next, a preparation example using citronella which has been known as essential oil as an oil solubilization additive of tranexamic acid is shown below.

To 4 mL of a screw-top sample tube were added 20 mg of tranexamic acid, 200 mg of citronella and 0.5 mL of ethanol, and the mixture was stirred for 1 day at room temperature. 200 mg of citronella was further added every other day, and the mixture was stirred for 3 days at room temperature. Three days later, 200 mg of citronella and 0.5 mL of ethanol were further added, and the mixture was stirred for 1 day at room temperature to prepare an ethanol solution. One day later, to 4 mL of a screw-top sample tube separately prepared were added 0.2 mL of the ethanol solution prepared above and 0.8 mL of IPM. As a result, a pale yellowish uniform solution was obtained.

From these results, it could be understood that tranexamic acid can be uniformly oil-solubilized in IPM by using essential oil containing citral or citronellal such as lemon grass and citronella.

### 5. Preparation example of percutaneously absorbable composition containing L-ascorbic acid oil-solubilized by terpene

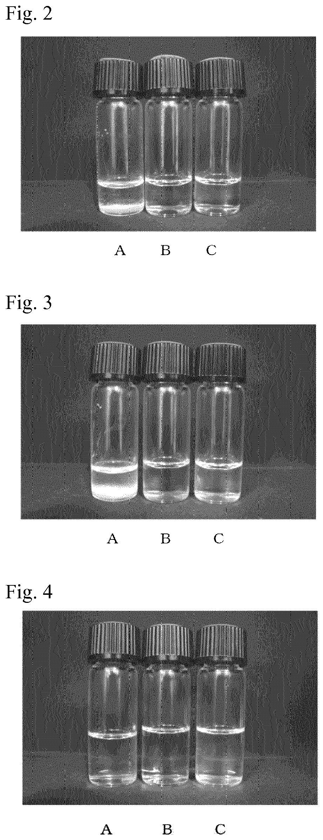

To 4 mL of a screw-top sample tube were added 10 mg of L-ascorbic acid, 100 mg of citral or (-)-citronellal, and 0.5 mL of ethanol, and the mixture was heated at 80°C for 1 hour. After heating, the mixture was allowed to cool for 5 minutes at room temperature, and 0.5 mL of IPM was added to prepare percutaneously absorbable compositions of colorless uniform solutions of Examples 7 and 8. The percutaneously absorbable composition (Example 7) to which citral was added and the percutaneously absorbable composition (Example 8) to which (-)-citronellal was added were colorless uniform solutions even at room temperature even after 16 hours (B (Example 7) and C (Example 8) in Fig. 2). On the other hand, in a solution (Comparative example 5) similarly prepared without adding these (citral or (-)-citronellal), a precipitate was observed with a lapse of time (A in Fig. 2).

From these results, it could be understood that L-ascorbic acid forms a complex with citral or (-)-citronellal and is uniformly oil-solubilized in IPM.

Further, preparation at room temperature was carried out.

To 9 mL of a screw-top sample tube were added 40 mg of L-ascorbic acid, 400 mg of citral and 2 mL of ethanol, and the mixture was stirred at room temperature for 12 hours. After 12 hours, 2 mL of IPM was added to the mixture to obtain a colorless uniform solution as in the case of heating preparation.

### 6. Preparation example of percutaneously absorbable composition containing L-ascorbic acid oil-solubilized by additive other than terpene

To 4 mL of a screw-top sample tube were added 40 mg of L-ascorbic acid, 200 mg of the additive shown in Table 3 and 1 mL of ethanol, and the mixture was heated at 80°C for 2 hours. After heating, the mixture was allowed to cool at room temperature for 5 minutes, and 1 mL of IPM was added to prepare percutaneously absorbable compositions of Examples 9 to 12.

The dispersed state of these Examples and Comparative examples was observed to confirm the presence or absence of oil solubilization. The results are shown in Table 3.

### [Table 3]

**Table 3 Addition effect of additive in oil-solubilization of L-ascorbic acid**

| | Example 9 | Example 10 | Example 11 | Example 12 |
|---|---|---|---|---|
| Kind of additive | Cinnamaldehyde | Vanillin | Dodecanal | 4-Butoxybenzaldehyde |
| Solution state | Colorless transparent | Colorless transparent | Colorless transparent | Colorless transparent |

From these results, it could be understood that L-ascorbic acid could be uniformly oil-solubilized in IPM by adding cinnamaldehyde, vanillin, dodecanal and 4-butoxybenzaldehyde, which have an aldehyde group and are used in food additives, etc., other than terpene.

### 7. Solubilization test of amino acid-citronellal or citral complex in ethanol

Solubilization of the complex with citronellal or citral in ethanol was examined while keeping the amino acid as crystal. The amino acids are different in the crystalline state in the commercial products depending on the kind thereof, so that there were fear that the results might cause fluctuation. Thus, an operation for enlarging the surface in contact with the liquid was added by making the difference due to crystals small as well as making the surface area as much as possible. Specifically, the crystals of the amino acids were ground in a mortar and then sieved through a 100 µm sieve to provide powders, which were used in the present test.

To 4 mL of a screw-top sample tube were added 20 mg of the amino acid powdered by the above-mentioned method and shown in Tables 4 and 5, and citronellal or citral in an amount from 700 mg to 900 mg at intervals of 50 mg to prepare samples. To the respective samples prepared was added 1 mL of ethanol, and the mixture was heated at 80°C for a maximum of 24 hours to obtain an ethanol solution of the amino acid-citronellal or citral complex. The amount and heating time of citronellal or citral necessary for solubilizing the obtained complex in ethanol, and the dispersed state of the obtained complex in ethanol was confirmed. The results are shown in Table 4 and

Table 5.

### [Table 4]

**Table 4 Solubilization investigation of amino acid in ethanol (at the time of using citronellal)**

| Amino acid | Lysine | Histidine | Serine | Threonine |
|---|---|---|---|---|
| Amount of citronellal (mg) | 700 | 700 | 700 | 700 |
| Heating time (h) | 0.5 | 17.5 | 3.5 | 3 |
| State of solution | Yellow uniform solution | Yellow uniform solution | Pale yellow uniform solution | Pale yellow uniform solution |
| Amino acid | Tryptophan | Glycine | L-cysteine | Proline |
| Amount of citronellal (mg) | 700 | 700 | 700 | 700 |
| Heating time (h) | 24 | 24 | 0.33 | 0.5 |
| State of solution | Yellow uniform solution | Yellow uniform solution | Pale yellow uniform solution | Yellow uniform solution |

### [Table 5]

**Table 5 Solubilization investigation of amino acid in ethanol (at the time of using citral)**

| Amino acid | Glutamic acid | Glutamine | L-Alanine | Valine |
|---|---|---|---|---|
| Amount of citral (mg) | 700 | 700 | 700 | 700 |
| Heating time (h) | 22.5 | 24 | 5 | 3 |
| State of solution | Reddish brown uniform solution | Reddish brown uniform solution | Reddish brown uniform solution | Reddish brown uniform solution |

| Amino acid | Isoleucine | Tyrosine | | |
|---|---|---|---|---|
| Amount of citral (mg) | 700 | 700 | | |
| Heating time (h) | 0.25 | 17 | | |
| State of solution | Reddish brown uniform solution | Reddish brown uniform solution | | |

From these results, it could be understood that various kinds of amino acids could be uniformly dissolved in ethanol by using citral or citronellal. Incidentally, the ethanol solution in which various amino acids were dissolved under the above conditions dissolves in IPM.

### 8. Preparation example of percutaneously absorbable composition containing minoxidil

To 4 mL of a screw-top sample tube were added 10 mg of minoxidil, 200 mg of citral or (-)-citronellal, and 0.5 mL of ethanol, and the mixture was heated at 80°C for 1 hour. After heating, the mixture was allowed to cool for 5 minutes at room temperature, and 0.5 mL of IPM was added to obtain a percutaneously absorbable composition. When the additive was citral, a yellow uniform solution (Example 13) was obtained, and when the additive was (-)-citronellal, a colorless uniform solution (Example 14) was obtained. Example 13 was a yellow uniform solution in IPM at room temperature even after 12 hours (B in Fig. 3). Similarly, Example 14 was a colorless uniform solution in IPM at room temperature even after 12 hours (C in Fig. 3). On the other hand, in a solution (Comparative example 6) prepared in the same manner without adding these (citral or (-)-citronellal), precipitate was observed with a lapse of time (A in Fig. 3).

From these results, it could be understood that minoxidil formed a complex with citral or (-)-citronellal and was uniformly oil-solubilized in IPM.

### 9. Preparation example of percutaneously absorbable composition containing peptide

Peptide A and Peptide B are peptides having an effect of treating hay fever. Investigation on oil solubilization regarding Peptide A or B was carried out using citral.

The oil solubilization experiment of Peptide A is as follows. To 1 mL of a screw-top sample tube were added 2 mg of Peptide A, 0.1 mL of citral and 0.1 mL of ethanol, and the mixture was heated at 80°C for 3 hours. After heating, the mixture was allowed to cool for 5 minutes at room temperature to prepare an ethanol solution. To separately prepared 1 mL of a screw-top sample tube was added 50 µL of the ethanol solution prepared above, and further 150 µL of IPM was added thereto to obtain a pale yellow peptide IPM solution.

In addition, the oil solubilization experiment of Peptide B was investigated on preparation at 80°C under heating and at room temperature.

Preparation conditions under heating at 80°C are as follows. To 1 mL of a screw-top sample tube were added 2 mg of Peptide B, 0.1 mL of citral and 0.1 mL of ethanol, and the mixture was heated at 80°C for 4 hours. After heating, the mixture was allowed to cool for 5 minutes at room temperature. To separately prepared 1 mL of a screw-top sample tube was added 50 µL of the prepared ethanol solution, and further 150 µL of IPM was added thereto to obtain a pale yellow peptide IPM solution.

Preparation conditions under stirring at room temperature are as follows. To 1 mL of a screw-top sample tube were added 2 mg of Peptide B, 0.1 mL of citral and 0.1 mL of ethanol, and the mixture was stirred at room temperature for 22 hours to obtain an ethanol solution. The ethanol solution after stirring became a yellow gel-like substance.

From these results, it could be understood that oil solubilization is achieved by adding citral to Peptides A and B.

### 10. Elucidation of structure of complex in solution by ESI-TOF-MASS

The structure of tranexamic acid complex in a solution was identified by using ESI-TOF-MASS. To 4 mL of a screw-top sample tube were added 40 mg of tranexamic acid, 400 mg of (-)-citronellal and 2 mL of ethanol, and the mixture was heated at 80°C for 1 hour. After heating, the mixture was allowed to cool for 5 minutes at room temperature, passed through a filter having a pore size of 0.45 µm, and the filtrate was obtained as an ethanol solution of tranexamic acid-(-)-citronellal complex, which was used for the measurement. Further, to another 4 mL of a screw-top sample tube were added 200 µL of the filtrate prepared above, and 1.8 mL of ethyl acetate or 1.8 mL of hexane, to prepare an ethyl acetate solution and a hexane solution of tranexamic acid-(-)-citronellal complex, respectively.

The ethanol solution of the tranexamic acid-(-)-citronellal complex was a yellow light solution (A in Fig. 4). In addition, the ethyl acetate solution and the hexane solution of the tranexamic acid-(-)-citronellal complex were all colorless and transparent (B and C in Fig. 4).

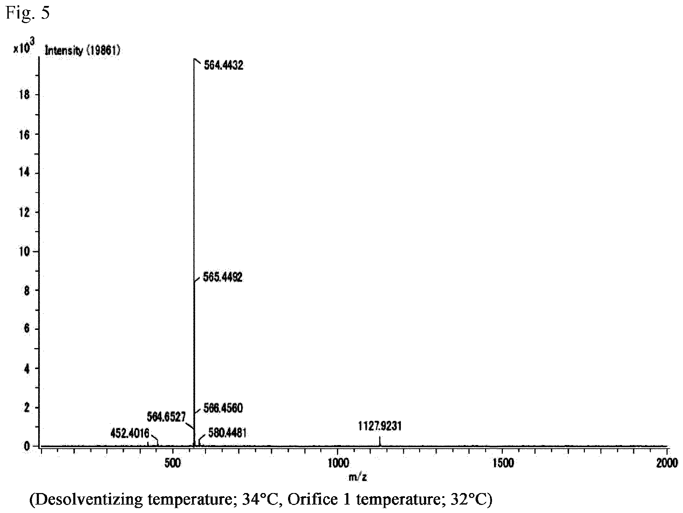

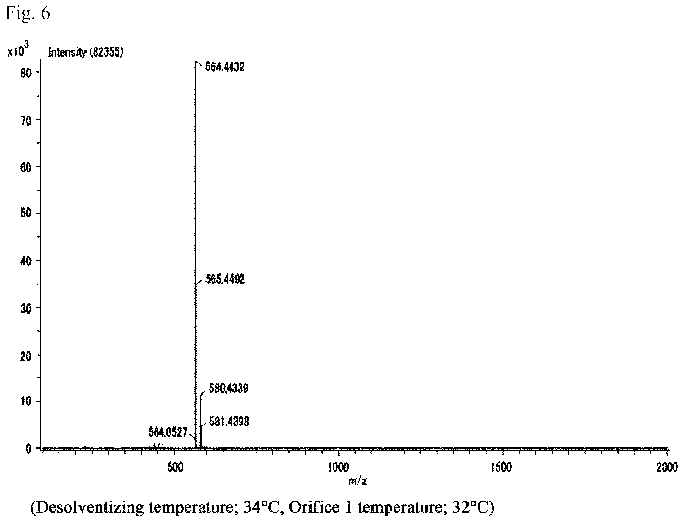

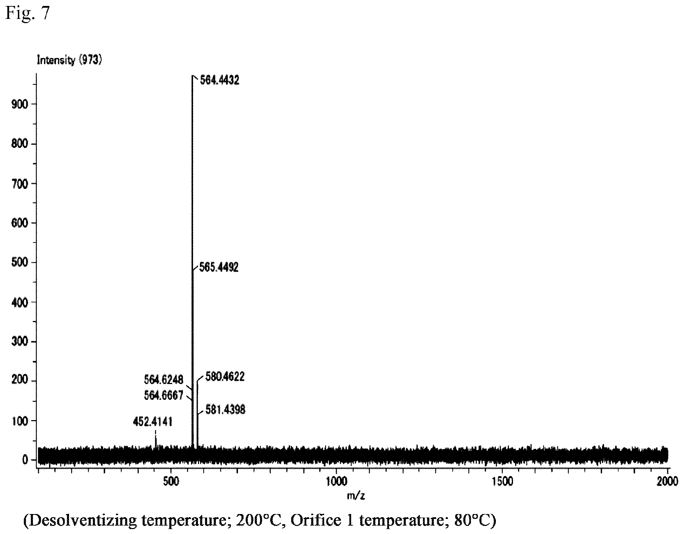

The conditions of ESI-TOF-MASS are as follows. Measurement conditions: ionization mode (ESI+), peak-to-peak voltage (2,000 V), orifice voltage (80V), desolventizing temperature (200°C or 34°C), orifice 1 temperature (80°C or 32°C). When the previously prepared ethanol solution of the tranexamic acid-(-)-citronellal complex was measured using ESI-TOF-MASS, a mass to charge ratio (m/z) of 564.44 was observed as the main peak. According to isotopic simulation, it was attributed to a complex consisting of one molecule of tranexamic acid, one molecule of citronellal and five molecules of ethanol (Fig. 5). Further, when the previously prepared ethyl acetate solution and hexane solution of the tranexamic acid-(-)-citronellal complex were measured by ESI-TOF-MASS, the same ion peak as the ethanol solution of the tranexamic acid-(-)-citronellal complex was confirmed, so that it was clarified that the complex was stably present even in these solutions (Figs. 6 and 7).

### 11. Experiment on releasability of oil-solubilized tranexamic acid into water

In order to confirm that the complex of tranexamic acid and citronellal releases tranexamic acid in water, an alcohol solution of a complex of tranexamic acid and citronellal (hereinafter also referred to as "tranexamic acid-citronellal complex") was added to an aqueous PBS solution, and an amount of the tranexamic acid in the aqueous PBS solution was determined by HPLC under stirring at room temperature.

Operating conditions of HPLC are that by using phosphate buffer (11.0 g of anhydrous sodium dihydrogen phosphate is dissolved in 500 mL of water, and 5 mL of triethylamine and 1.4 g of sodium dodecyl sulfate are added thereto. After adjusting a pH to 2.5 by adding phosphoric acid, the mixture is diluted to 600 mL with pure water) and methanol with 60/40 (vol/vol) as a mobile phase, it was passed through a column (Inertsil ODS-3 manufactured by GL Science) set at 25°C with a flow rate of 0.7 mL/min, and the peak of the tranexamic acid was detected by a detection wavelength of 220 nm. A calibration curve was prepared in the range of 0.1 mg/mL to 10 mg/mL, and the amount of the tranexamic acid in the releasability experiment was quantified.

The releasability test was carried out as follows. Incidentally, the aqueous PBS solution used in this experiment was a 10-fold diluted 10×D-PBS (-) with pure water. To 4 mL of a screw-top sample tube were added 10 mg of tranexamic acid, 100 mg of citronellal and 0.5 mL of alcohol shown in Table 6, and the mixture was heated at 80°C for 1 hour. After heating, the mixture was allowed to cool at room temperature to obtain an alcohol solution of tranexamic acid-citronellal complex. 200 µL of this alcohol solution was added dropwise to 5 mL of an aqueous PBS solution, and the mixture was stirred at room temperature. The aqueous PBS solution after 30 minutes and one day were sampled, and an amount of the tranexamic acid in the aqueous PBS solution was quantified by HPLC. The results are shown in Table 6.

### [Table 6]

**Table 6 Released ratio of tranexamic acid**

| Alcohol | After 30 minutes | After one day |
|---|---|---|
| Methanol | 70% | 90% |
| Ethanol | 27% | 30% |
| Geraniol | 10% | 18% |

From these results, it could be understood that more tranexamic acid was released from the tranexamic acid-citronellal complex into the aqueous PBS solution as the carbon chain length of the alcohol was shorter. In addition, it could be also understood that the tranexamic acid was gradually released from after 30 minutes to even after one day. This suggests that there is a possibility that the release rate can be controlled by appropriately selecting the percutaneous absorption controller.

### 12. Experiment on releasability of oil-solubilized ascorbic acid into water

In order to confirm that the complex of L-ascorbic acid and citral releases L-ascorbic acid in water, an alcohol solution of a complex of L-ascorbic acid and citral (hereinafter also referred to as "ascorbic acid-citral complex") was added to an aqueous PBS solution, and an amount of the L-ascorbic acid in the aqueous PBS solution was determined by using F kit L-ascorbic acid (manufactured by Roche/R-Biopharm) under stirring at room temperature.

The releasability test was carried out as follows. Incidentally, the aqueous PBS solution used in this experiment was a 10-fold diluted 10×D-PBS(-) with pure water. To 8 mL of a screw-top sample tube were added 40 mg of L-ascorbic acid, 400 mg of citral and 2 mL of ethanol, and the mixture was stirred for 12 hours at room temperature. After 12 hours, 2 mL of IPM was added to the mixture to obtain an IPM solution of an ascorbic acid-citral complex. 200 µL of this IPM solution was added dropwise to 5 mL of an aqueous PBS solution, and the mixture was stirred at room temperature. The aqueous PBS solution after 24 hours was sampled and an amount of the L-ascorbic acid in the aqueous PBS solution was quantified with F kit L-ascorbic acid. The absorbance was measured with a microplate reader. As a result, it could be understood that 83% of L-ascorbic acid was released.

### 13. Percutaneous absorption test of oil-solubilized tranexamic acid

The stratum corneum is hydrophobic and becomes more hydrophilic as it is deeper. That is, in order to penetrate the barrier of the stratum corneum, oil solubilization is an effective means. In the test of item 11, it was clarified that the alcohol solution of the tranexamic acid-citronellal complex releases the tranexamic acid in the aqueous PBS solution. Thus, by using the ethanol solution of the tranexamic acid-citronellal complex, a percutaneous absorption test was carried out to determine whether or not to release the tranexamic acid in the aqueous PBS solution after breaking through the skin barrier.

The percutaneous absorption test was carried out as follows. Incidentally, the aqueous PBS solution used in this experiment was a 10-fold diluted 10×D-PBS(-) with pure water. A stirring bar and 5 mL of the aqueous PBS solution were added to a receiver phase of Franz cell, a membrane for the percutaneous absorption test (Strat-M membrane (manufactured by Merck Millipore)) was sandwiched in an upper part of the aqueous PBS solution, and water of 32°C was passed through the water jacket portion of the Franz cell. 200 µL of an aqueous tranexamic acid solution or an ethanol solution of the tranexamic acid-citronellal complex was placed on a membrane for the percutaneous absorption test, and the receiver phase was stirred. After 24 hours, the aqueous PBS solution was sampled, and an amount of the tranexamic acid was quantified by HPLC.

As a result, in the case of the aqueous tranexamic acid solution, the peak of the tranexamic acid in the aqueous PBS solution of the receiver phase was 0.3%. On the other hand, it could be confirmed that in the ethanol solution of the tranexamic acid-citronellal complex, 57% of the tranexamic acid was released into the aqueous PBS solution of the receiver phase.

Further, when the experiment of the tranexamic acid-citronellal complex IPM solution was carried out in the same manner, percutaneous absorbability of 7% was confirmed in the solution prepared under heating at 80°C, and percutaneous absorbability of 9% was confirmed in the solution prepared at room temperature.

From these results, it was shown that the tranexamic acid-citronellal complex had percutaneous absorbability, and was promising as a percutaneous drug delivery system (DDS) material.

### 14. Percutaneous absorption test of oil-solubilized L-ascorbic acid

As in the case of the tranexamic acid, in the test of the item 12, it was clarified that an IPM solution of the ascorbic acid-citral complex releases L-ascorbic acid in the aqueous PBS solution. Thus, by using the ascorbic acid-citral complex IPM solution, a percutaneous absorption test was carried out to determine whether or not to release the L-ascorbic acid in the aqueous PBS solution after breaking through the skin barrier.

As a percutaneous absorption test, pig skin (Yucatan Micro Pig (manufactured by Charles River Laboratories Japan, Inc.)) was used. Incidentally, the aqueous PBS solution used in this experiment was a 10-fold diluted 10×D-PBS(-) with pure water. A stirring bar and 5 mL of the aqueous PBS solution were added to a receiver phase of Franz cell, the pig skin from which fat had been removed was sandwiched in an upper part of the aqueous PBS solution, and water of 32°C was passed through the water jacket portion of the Franz cell. 200 µL of an aqueous L-ascorbic acid solution or an IPM solution of an ascorbic acid-citral complex was placed on the pig skin, and the receiver phase was stirred. After 24 hours, the pig skin was treated as follows. The surface of the pig skin was washed with 1 mL of the aqueous PBS solution/methanol/ acetonitrile (2/1/1 (vol/vol/vol)) four times, the portion which was in contact with the receiver layer was cut into 8 equal parts, and charged in 1.5 mL of a microtube. Thereto was added 500 µL of the aqueous PBS solution/methanol/acetonitrile (2/1/1 (vol/vol/vol)), the mixture was shaken by a vortex mixer for 1 hour, and 100 µL of the aqueous PBS solution/methanol/acetonitrile extract in the microtube was used as a quantitative solution of L-ascorbic acid. 4 mL of the receiver layer was collected, lyophilized for 1 day, and concentrated. To the residue after concentration was added 500 µL of the aqueous PBS solution, and after dissolving the residue, 200 µL of which was used as a quantitative solution. As a quantitative determination of L-ascorbic acid, F kit L-ascorbic acid was used, and the absorbance was measured with a microplate reader.

As a result, in the case of the aqueous L-ascorbic acid solution, the detection limit or less of a permeation amount was shown in the pig skin and the receiver layer, but in the case of the IPM solution of the ascorbic acid-citral complex, 84.5 µg/cm² of a permeation amount was confirmed in the pig skin, and 15.2 µg/cm² of a permeation amount in the receiver layer.

From these results, it was shown that the ascorbic acid-citral complex had percutaneous absorbability, and was promising as a percutaneous drug delivery system (DDS) material.

### 15. Percutaneous absorption test of oil-solubilized L-ascorbic acid using additives other than citral

A percutaneous absorption test was carried out when an additive other than citral was used as an additive for oil-solubilizing L-ascorbic acid.

As a percutaneous absorption test, pig skin (Yucatan Micro Pig (manufactured by Charles River Laboratories Japan, Inc.)) was used. Incidentally, the aqueous PBS solution used in this experiment was a 10-fold diluted 10×D-PBS(-) with pure water. A stirring bar and 5 mL of the aqueous PBS solution were added to a receiver phase of Franz cell, the pig skin from which fat had been removed was sandwiched in an upper part of the aqueous PBS solution, and water of 32°C was passed through the water jacket portion of the Franz cell. 200 µL of an aqueous L-ascorbic acid solution or an IPM solution of an oil-solubilized L-ascorbic acid was placed on the pig skin, and the receiver phase was stirred. After 24 hours, the pig skin was treated as follows. The surface of the pig skin was washed with 1 mL of the aqueous PBS solution/methanol/ acetonitrile (2/1/1 (vol/vol/vol)) four times, the portion which was in contact with the receiver layer was cut into 8 equal parts, and charged in 1.5 mL of a microtube. To the mixture was added 500 µL of the aqueous PBS solution/methanol/acetonitrile (2/1/1 (vol/vol/vol)), the mixture was shaken by a vortex mixer for 1 hour, and 100 µL of the aqueous PBS solution/methanol/acetonitrile extract in the microtube was used as a quantitative solution of L-ascorbic acid. 4 mL of the receiver layer was collected, lyophilized for 1 day, and concentrated. To the residue after concentration was added 500 µL of the aqueous PBS solution, and after dissolving the residue, 200 µL of which was used as a quantitative solution. As a quantitative determination of L-ascorbic acid, F kit L-ascorbic acid was used, and the absorbance was measured with a microplate reader. The results are shown in Table 7.

### [Table 7]

**Table 7 Percutaneous absorbability of oil-solubilized L-ascorbic acid using additives other than citral**

| | Example 15 | Example 16 | Example 17 |
|---|---|---|---|
| Kind of additive | Cinnamylaldehyde | Vanillin | Dodecanal |
| Permeation amount to pig skin (µg/cm²) | 15.2 | 74.4 | 26.9 |
| Permeation amount to receiver layer (µg/cm²) | 122.5 | 59.0 | 40.5 |

From these results, it was shown that the IPM solution of the oil-solubilized L-ascorbic acid using additives other than citral had percutaneous absorbability, and was promising as a percutaneous drug delivery system (DDS) material.

### UTILIZABILITY IN INDUSTRY

The complex of the present invention can improve percutaneous absorbability of the water-soluble effective ingredient to increase the content thereof in the skin, and can accomplish medical effect by releasing the water-soluble effective ingredient according to water and acidic conditions inside the skin, so that the percutaneously absorbable composition containing such a complex of the present invention can be utilized for the external preparation for skin, for example, medicines used for external skin therapy and cosmetics.

In addition, release of the water-soluble effective ingredient from the complex of the present invention inside the skin can be controlled by appropriately using a percutaneous absorption controller, so that it can be utilized in a drug delivery system.

## Claims

1. A complex formed by a water-soluble effective ingredient and a fat-soluble compound having an aldehyde group through an interaction which can be dissociated in water.

2. The complex according to Claim 1, wherein the fat-soluble compound having an aldehyde group is at least one selected from the group consisting of terpene having an aldehyde group, lignoid having an aldehyde group and vanilloid having an aldehyde group.

3. The complex according to Claim 2, wherein the terpene having an aldehyde group is citral or citronellal.

4. The complex according to Claim 2, wherein the lignoid having an aldehyde group is cinnamaldehyde.

5. The complex according to Claim 2, wherein the vanilloid having an aldehyde group is vanillin.

6. The complex according to any one of Claims 1 to 5, wherein the water-soluble effective ingredient is a compound having an amino group, a hydroxyl group or a thiol group.

7. A percutaneously absorbable composition which comprises the complex according to any one of Claims 1 to 6, a percutaneous absorption controller and a fat-soluble medium.

8. The percutaneously absorbable composition according to Claim 7, wherein the complex is dissolved in the fat-soluble medium.

9. The composition according to Claim 7 or 8, wherein the fat-soluble medium is a percutaneous absorption promotable fat-soluble medium.

10. The composition according to Claim 9, wherein the percutaneous absorption promotable fat-soluble medium is at least one selected from the group consisting of isopropyl myristate, squalane, squalene, silicone oil, jojoba oil, almond oil, olive oil, horse oil and mineral oil.

11. A composition which comprises a water-soluble effective ingredient, a fat-soluble compound having an aldehyde group and a percutaneous absorption controller.

12. The composition according to Claim 11, which further comprises a fat-soluble medium.

13. The composition according to Claims 7 to 12, wherein the percutaneous absorption controller is an alcohol.

14. The composition according to Claim 13, wherein the alcohol is at least one selected from the group consisting of methanol, ethanol, geraniol, isopropyl alcohol, and glycerol.

15. A method for controlling release of the water-soluble effective ingredient from the complex according to any one of Claims 1 to 6 in skin which comprises a step of dissolving the complex in a percutaneous absorption controller.

16. A method for releasing the water-soluble effective ingredient from the complex according to any one of Claims 1 to 6 in skin which comprises a step of dissolving the complex in a percutaneous absorption controller.

17. A method for releasing a water-soluble effective ingredient in skin from a composition containing the water-soluble effective ingredient, a fat-soluble compound having an aldehyde group and a percutaneous absorption controller.

18. A method for producing a complex formed by a water-soluble effective ingredient and a fat-soluble compound having an aldehyde group through an interaction which can be dissociated in water, which comprises a step of mixing powder of the water-soluble effective ingredient and the fat-soluble compound having an aldehyde group.

19. A method for producing a composition containing a water-soluble effective ingredient, a fat-soluble compound having an aldehyde group and a percutaneous absorption controller, which comprises a step of mixing powder of the water-soluble effective ingredient, the fat-soluble compound having an aldehyde group and the percutaneous absorption controller.

20. The complex according to Claim 1, wherein the fat-soluble compound having an aldehyde group is contained in essential oil.

21. The complex according to Claim 20, wherein the essential oil is lemon grass or citronella.
